# EUROPEAN PATENT APPLICATION

(11) **EP 2 305 316 A2**
(43) Date of publication of application: **06.04.2011**
(21) Application number: 10187178.8
(22) Date of filing: 07.12.2006
(51) Int. Cl.: A61K 49/14, A61K 51/08

(54) **Diphosphorylated glycopeptide imaging agent for fibrosis**

(30) Priority: 08.12.2005 GB 0524987
(62) Divisional of application: 06831370.9
(71) Applicant: GE Healthcare Limited, Buckinghamshire HP8 4SP (GB)
(72) Inventor: Chettibi, Salah, Amersham Buckinghamshire HP7 9LL (GB); Newton, Ben, Amersham Buckinghamshire HP7 9LL (GB); Guilbert, Benedicte, Amersham Buckinghamshire HP7 9LL (GB); Tolleshaug, Helge, 0588 Oslo (NO); Solbakken, Magne, 0401 Oslo (NO)
(74) Representative: Bannan, Sally

(57) **Abstract**

The present invention provides a novel imaging agent suitable for the non-invasive visualization of fibrosis. A method for the preparation of the imaging agent is also provided by the invention, as well as a precursor for use in said method. The invention also provides a pharmaceutical composition comprising the imaging agent and a kit for the preparation of the pharmaceutical composition. In a further aspect, use of the imaging agent for *in vivo* imaging and in the preparation of a medicament for the diagnosis of a condition in which the mannose-6-phosphate receptor is upregulated is provided.

## Description

### Technical Field of the Invention

The present invention relates to diagnostic imaging and in particular to the diagnostic imaging of fibrosis. Diagnostic imaging agents are described which are suitable for this purpose, particularly for the diagnostic imaging of fibrosis in the liver, heart, kidneys and lungs.

### Description of Related Art

Fibrosis is a process characterized by the excessive secretion of extracellular matrix components. This is caused by increased synthesis and decreased degradation of matrix proteins, most notably collagen types I and III, and is triggered as a response to tissue damage resulting from inflammation, infection or injury. In simple terms, fibrosis is scar tissue and forms part of all "repair" processes in tissue. However, because of ongoing inflammation, infection and repeated injury, fibrosis scar tissue builds up and does not replace "functional" cells, thus leading to abnormal organ function and eventually organ failure.

Fibrosis is one of the major, classic pathological processes in medicine. It is a key component of multiple diseases that affect millions of people worldwide including:
a) Lung diseases such as idiopathic pulmonary fibrosis (lung fibrosis of unknown origin), asthma and chronic obstructive pulmonary disease
b) Scleroderma: a heterogeneous and life threatening disease characterised by the excessive extracellular matrix deposition within connective tissue of the body (i.e. skin and visceral organs)
c) Post-surgical scarring following transplantation
d) Diabetic retinopathy and age-related macular degeneration (fibrotic diseases of the eye and leading causes of blindness)
e) Cardiovascular disease including atherosclerosis and vulnerable plaque.
f) Kidney fibrosis linked to diabetes - diabetic nephropathy and glomerulosclerosis
g) IgA nephropathy (causes of kidney failure and the need for dialysis and retransplant)
h) Cirrhosis and biliary atresia (leading causes of liver fibrosis and failure)
i) Rheumatoid arthritis
j) Autoimmune diseases such as dermatomyositis
k) Congestive heart failure

The clinical manifestations of fibrosis vary widely. Taking the example of cirrhosis, the clinical manifestations vary from no symptoms at all, to liver failure, and are determined by both the nature and severity of the underlying liver disease as well as the extent of hepatic fibrosis. Up to 40% of patients with cirrhosis are asymptomatic and may remain so for more than a decade, but progressive deterioration is inevitable once complications develop including ascites, variceal hemorrhage or encephalopathy. Fibrosis and cirrhosis therefore represent the consequences of a sustained wound healing response to chronic liver injury from a variety of causes including viral, autoimmune, drug induced, cholestatic and metabolic diseases. The common causes of liver fibrosis and cirrhosis include immune mediated damage, genetic abnormalities, and non-alcoholic steatohepatitis (NASH), which is particularly associated with diabetes and metabolic syndrome (MS). There is a high incidence of MS in the western population. This syndrome typically occurs in individuals who are obese, have hyperlipidemia and hypertension, and often leads to the development of type II diabetes. The hepatic manifestation of metabolic syndrome is non-alcoholic fatty liver disease (NAFLD), with an estimated prevalence in the USA of 24% of the population. A fatty liver represents the less severe end of a spectrum of NAFLD that may progress to NASH and ultimately to cirrhosis of the liver. The development of fibrosis demonstrates a risk of such progression, and is presently assessed by means of a liver biopsy. However, liver biopsy causes significant discomfort, is not without risk and is costly. Furthermore, available blood tests for hepatic fibrosis are not reliable in NAFLD.

WO 00/23113 discloses a compound comprising a peptide and a carrier molecule, where the peptide targets hepatic stellate cells (HSC). Examples of target receptors presented are those specific for HSC or upregulated on HSC during disease (including fibrotic disease), e.g. platelet-derived growth factor receptor, collagen VI receptor, transforming growth factorβ receptor, interleukin-1β receptor and tumour necrosis factorα receptor. In another aspect presented, the target receptor is the cation-independent mannose-6-phosphate (M6P) receptor, which has been reported to be over-expressed in liver fibrosis. Examples of suitable carriers are human serum albumin, proteins, polymeric carriers and liposomes. The compounds disclosed are primarily drug carriers which can be used to target all kinds of therapeutic agents. It is stated that the carrier is suitably larger than 5000 Da. It is also mentioned that they may be applied for the visualisation of HSC for diagnostic purposes, stating that the compounds may further comprise a diagnostic marker. However, there is no description of how a diagnostic marker might be attached to the disclosed compounds.

EP 1495769 A1 discloses glycoside-compound conjugates for use in antisense strategies, particularly *in vivo* antisense strategies. The conjugates comprise a glycoside linked to a compound, in which glycoside is a ligand capable of binding to a mannose-6-phosphate receptor of a muscle cell. Conjugates which are "marked" to include fluorescent, radioactive, enzymatic or molecular markers are mentioned for *in vivo* or *in vitro* diagnosis, but there is no description of how to obtain such marked compounds.

A need therefore exists for an alternative non-invasive test for the detection of fibrosis and in particular liver fibrosis.

### Summary of the Invention

The present invention provides a novel imaging agent suitable for the non-invasive visualization of fibrosis. A method for the preparation of the imaging agent is also provided by the invention, as well as a precursor for use in said method. The invention also provides a pharmaceutical composition comprising the imaging agent and a kit for the preparation of the pharmaceutical composition. In a further aspect, use of the imaging agent for *in vivo* imaging and in the preparation of a medicament for the diagnosis of a condition in which the mannose-6-phosphate receptor is upregulated is provided.

### Detailed Description of the Invention

In one aspect, the present invention provides an imaging agent comprising:
(i) a vector with affinity for the mannose-6-phosphate (M6P) receptor; and,
(ii) an imaging moiety.
wherein the imaging moiety is present either as an integral part of the vector or the imaging moiety is conjugated to the vector *via* a suitable chemical group.

In the context of the present invention, the term "M6P receptor" specifically relates to the extracellular domain of the cation-independent M6P receptor.

By the term "imaging agent" is meant a compound designed to target a particular physiology or pathophysiology in a mammal, and which can be detected following its administration to the mammalian body *in vivo.*

As stated above, in the imaging agent of the invention, the imaging moiety may be present as an integral part of the vector, e.g. one of the atoms of the vector could be ¹¹C instead of ¹²C. Alternatively, the imaging moiety may be conjugated to the vector *via* a suitable chemical group, e.g. a metal chelate which can complex an imaging moiety which is a metal ion. A linker may also be present linking the vector to either the suitable chemical group or directly to the imaging moiety itself. Suitable linkers of the present invention are of Formula -(L¹)ₙ- wherein:
each L¹ is independently -CO-, -CR₂-, -CR=CR-, -C≡C-, -CR₂CO₂-, -CO₂CR₂-, -NR-, - NRCO-, -CONR-, -NR(C=O)NR-, -NR(C=S)NR-, -SO₂NR-, -NRSO₂-, -CR₂OCR₂-, - CR₂SCR₂-, -CR₂NRCR₂-, a C₄₋₈ cycloheteroalkylene group, a C₄₋₈ cycloalkylene group, a C₅₋₁₂ arylene group, a C₃₋₁₂ heteroarylene group, an amino acid residue, a polyalkyleneglycol, polylactic acid or polyglycolic acid moiety;
n is an integer of value 0 to 20;
each R group is independently H or C₁₋₁₀ alkyl, C₃₋₁₀ alkylaryl, C₂₋₁₀ alkoxyalkyl, C₁₋₁₀ hydroxyalkyl, C₁₋₁₀ fluoroalkyl, or 2 or more R groups, together with the atoms to which they are attached form a carbocyclic, heterocyclic, saturated or unsaturated ring.

It is envisaged that branched linker groups are also possible, i.e. the linker group -(L¹)ₙ-substituted with a further linker -(L²)ₒ-R', wherein L², o and R' are as defined respectively for L¹, n a nd R a bove.

Such linkers are particularly useful in the context of manipulating the biodistribution and/or excretion profiles of the imaging agent. For example, the inclusion of a linker comprising polyethylene glycol groups or acetyl groups can improve the blood residence time of the imaging agent.

By the term "amino acid" is meant an L- or D-amino acid, amino acid analogue (e.g. napthylalanine) or amino acid mimetic which may be naturally occurring or of purely synthetic origin, and may be optically pure, i.e. a single enantiomer and hence chiral, or a mixture of enantiomers. Preferably the amino acids of the present invention are optically pure.

Such linkers also have application in relation to other parts of the invention as described below. For the present application, preferred L¹ and L² groups are -CO-, -CH₂-, -NH-, -NHCO-, -CONH-, -CH₂OCH₂-, and amino acid residues.

The term "affinity" in the context of the present invention is taken to mean binding to the M6P receptor *in vitro* with a Kd value of less than 100nM, preferably less than 50nM and most preferably less than 10nM. Alternatively, affinity may be defined as the ability to inhibit binding of β-galactosidase to M6P receptor *in vitro* [described by Distler et al 1991 J. Biol. Chem. 266(32) 21687-92], wherein IC₅₀ values of less than 10µM, preferably less than 1µM, most preferably less than 0.1µM and especially preferably less than 0.01µM. Affinity may also be defined as the ability to inhibit binding of Insulin like Growth Factor-II (IGF-II) to M6P receptor *in vitro* [Marron P.G. et al 1998 J. Biol. Chem. 273(35) 22358-22366], wherein IC₅₀ values of less than 10µM, preferably less than 1µM, most preferably less than 0.1µM and especially preferably less than 0.01µM.

The imaging agent of the present invention preferably does not have conjugated to the M6P vector an oligonucleotide, RNA, DNA, peptide nucleic acid, growth factor, vaccine, vitamin, or antibody. Most preferably imaging agent of the present invention has only the imaging moiety conjugated to the M6P vector, i.e. nothing further is conjugated to the M6P vector.

The vector for the M6P receptor comprises a diphosphorylated glycopeptide.

WO 95/014036 discloses diphosphorylated glycopeptides such compounds which bind to M6P receptor and are useful in the treatment of inflammatory and other diseases and their synthesis is described by Christensen et al (J. Chem. Soc. Perkin Trans. 1994 1299-1310). Any of these compounds are suitable vectors for the present invention. They may be represented by Formula I: 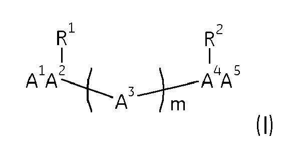 wherein
R¹ and R² are independently selected from
(i) a natural L- or D-monosaccharide chosen from: glucose, mannose, galactose, fucose, rhammanose, N-acetylglucosamine, N-acetylgalactosaminyl, fructose and N-acetylneuraminic acid, or phosphorylated or sulphated versions thereof; or,
(ii) an oligosaccharide composed of monosaccharides selected from (i);

A¹ and A⁵ are independently selected from the group consisting of -H, -OH, -NH₂, -acetyl, D-or L-amino acids, peptides, glycopeptides, peptidomimetics and oligonucleotides,

A² and A⁴ are independently selected from the group of D- or L-hydroxy amino acids, e.g. Ser, Thr, Hyl, Hyp, Tyr or D- or L-carboxamido amino acids, e.g. Asn and Gln, and

A³ is selected from the group of genetically encoded or non-encoded amino acids in their D-or L-form or peptidomimetics or nucleotides, and wherein m is an integer between 1 and 30 and wherein any residue in the linear sequence A¹ - A⁵ may be covalently linked to form a cyclic compound.

Preferably, R¹ and R² are M6P groups, A¹ is an acetyl group and A⁵ is -NH₂, A² and A⁴ are Thr, and A³ is a chain of between 1 and 5 amino acid residues.

Most preferred diphosphorylated glycopeptides of the present invention are:
(i) Ac-Thr[α-D-M6P-(1,2)-α-D-mannose]-Lys(aminobenzamide)-Thr[α-D-M6P-(1,2)-α-D-mannose]-NH₂ ("Glycopeptide Compound 1")
(ii) i) Ac-Thr[α-D-M6P]-Gly-Lys-Gly-Thr[α-D-M6P]-NH₂ ("Glycopeptide Compound 2")

Where a diphosphorylated glycopeptide is the vector with affinity for the M6P receptor, the imaging moiety is preferably conjugated *via* one of the amino acids present in the group (A³)m.

In an alternative preferred embodiment, the diphosphorylated glycopeptide can be one of the above-described compounds but wherein one or more of the phosphate groups is replaced with a phosphonate group.

It is furthermore envisaged that the vector may be a multivalent targeting vector combining two or more of the above-described vectors. Imaging agents comprising such vectors are anticipated to exhibit an increased affinity for M6P receptor due to the fact that M6P receptor contains independent binding sites for each of the vectors. Following administration of the imaging agent, the "imaging moiety" may be detected either external to the human body or *via* use of detectors designed for use *in vivo,* such as intravascular radiation or optical detectors such as endoscopes, or radiation detectors designed for intraoperative use.

The imaging moiety is preferably chosen from:
(i) a radioactive metal ion;
(ii) a paramagnetic metal ion;
(iii) a gamma-emitting radioactive halogen;
(iv) a positron-emitting radioactive non-metal;
(v) a hyperpolarised NMR-active nucleus;
(vi) a reporter suitable for *in vivo* optical imaging;
(vii) a β-emitter suitable for intravascular detection.

When the imaging moiety is a radioactive metal ion, i.e. a radiometal, suitable radiometals can be either positron emitters such as ⁶⁴Cu, ⁴⁸V, ⁵²Fe, ⁵⁵Co, ^{94m}Tc or ⁶⁸Ga; γ-emitters such as ^{99m}Tc, ¹¹¹In ^{113m}In, or ⁶⁷Ga. Preferred radiometals are ^{99m}Tc, ⁶⁴Cu, ⁶⁸Ga and ¹¹¹In. Most preferred radiometals are γ-emitters, especially ^{99m}Tc.

When the imaging moiety is a paramagnetic metal ion, suitable such metal ions include: Gd(III), Mn(II), Cu(II), Cr(III), Fe(III), Co(II), Er(II), Ni(II), Eu(III) or Dy(III). Preferred paramagnetic metal ions are Gd(III), Mn(II) and Fe(III), with GD(III) being especially preferred.

When the imaging moiety is a gamma-emitting radioactive halogen, the radiohalogen is suitably chosen from ¹²³I, ¹³¹I or ⁷⁷Br. ¹²⁵I is specifically excluded as it is not suitable for use as an imaging moiety for diagnostic imaging. A preferred gamma-emitting radioactive halogen is ¹²³I.

When the imaging moiety is a positron-emitting radioactive non-metal, suitable such positron emitters include: ¹¹C, ¹³N, ¹⁵O, ¹⁷F, ¹⁸F, ⁷⁵Br, ⁷⁶Br or ¹²⁴I. Preferred positron-emitting radioactive non-metals are ¹¹C, ¹³N, ¹⁸F and ¹²⁴I, especially ¹¹C and ¹⁸F, most especially ¹⁸F.

When the imaging moiety is a hyperpolarised NMR-active nucleus, such NMR-active nuclei have a non-zero nuclear spin, and include ¹³C, ¹⁵N, ¹⁹F, ²⁹Si and ³¹P. Of these, ¹³C is preferred. By the term "hyperpolarised" is meant enhancement of the degree of polarisation of the NMR-active nucleus over its' equilibrium polarisation. The natural abundance of ¹³C (relative to ¹²C) is about 1%, and suitable ¹³C-labelled compounds are suitably enriched to an abundance of at least 5%, preferably at least 50%, most preferably at least 90% before being hyperpolarised. At least one carbon atom of the imaging agent of the invention is suitably enriched with ¹³C, which is subsequently hyperpolarised.

When the imaging moiety is a reporter suitable for *in vivo* optical imaging, the reporter is any moiety capable of detection either directly or indirectly in an optical imaging procedure. The reporter might be a light scatterer (e.g. a coloured or uncoloured particle), a light absorber or a light emitter. More preferably the reporter is a dye such as a chromophore or a fluorescent compound. The dye can be any dye that interacts with light in the electromagnetic spectrum with wavelengths from the ultraviolet light to the near infrared. Most preferably the reporter has fluorescent properties.

Preferred organic chromophoric and fluorophoric reporters include groups having an extensive delocalized electron system, e.g. cyanines, merocyanines, indocyanines, phthalocyanines, naphthalocyanines, triphenylmethines, porphyrins, pyrilium dyes, thiapyrilium dyes, squarylium dyes, croconium dyes, azulenium dyes, indoanilines, benzophenoxazinium dyes, benzothiaphenothiazinium dyes, anthraquinones, napthoquinones, indathrenes, phthaloylacridones, trisphenoquinones, azo dyes, intramolecular and intermolecular charge-transfer dyes and dye complexes, tropones, tetrazines, *bis*(dithiolene) complexes, *bis*(benzene-dithiolate) complexes, iodoaniline dyes, *bis*(S,O-dithiolene) complexes. Fluorescent proteins, such as green fluorescent protein (GFP) and modifications of GFP that have different absorption/emission properties are also useful. Complexes of certain rare earth metals (e.g., europium, samarium, terbium or dysprosium) are used in certain contexts, as are fluorescent nanocrystals (quantum dots).

Particular examples of chromophores which may be used include: fluorescein, sulforhodamine 101 (Texas Red), rhodamine B, rhodamine 6G, rhodamine 19, indocyanine green, Cy2, Cy3, Cy3.5, Cy5, Cy5.5, Cy7, Marina Blue, Pacific Blue, Oregon Green 88, Oregon Green 514, tetramethylrhodamine, and Alexa Fluor 350, Alexa Fluor 430, Alexa Fluor 532, Alexa Fluor 546, Alexa Fluor 555, Alexa Fluor 568, Alexa Fluor 594, Alexa Fluor 633, Alexa Fluor 647, Alexa Fluor 660, Alexa Fluor 680, Alexa Fluor 700, and Alexa Fluor 750.

Particularly preferred are dyes which have absorption maxima in the visible or near infrared (NIR) region, between 400 nm and 3 µm, particularly between 600 and 1300 nm. Optical imaging modalities and measurement techniques include, but not limited to: luminescence imaging; endoscopy; fluorescence endoscopy; optical coherence tomography; transmittance imaging; time resolved transmittance imaging; confocal imaging; nonlinear microscopy; photoacoustic imaging; acousto-optical imaging; spectroscopy; reflectance spectroscopy; interferometry; coherence interferometry; diffuse optical tomography and fluorescence mediated diffuse optical tomography (continuous wave, time domain and frequency domain systems), and measurement of light scattering, absorption, polarisation, luminescence, fluorescence lifetime, quantum yield, and quenching.

When the imaging moiety is a β-emitter suitable for intravascular detection, suitable such β-emitters include the radiometals ⁶⁷Cu, ⁸⁹Sr, ⁹⁰Y, ¹⁵³Sm, ¹⁸⁶Re, ¹⁸⁸Re or ¹⁹²Ir, and the non-metals ³²P, ³³P, ³⁸S, ³⁸Cl, ³⁹Cl, ⁸²Br and ⁸³Br.

Preferred imaging moieties are those which can be detected externally in a non-invasive manner following administration *in vivo.* Most preferred imaging moieties are radioactive, especially radioactive metal ions, gamma-emitting radioactive halogens and positron-emitting radioactive non-metals, particularly those suitable for imaging using SPECT or PET. The various means by which these imaging moieties can be incorporated into each of the vector types are outlined below in the description of a further aspect of the invention.

Preferred imaging agents of the invention do not undergo facile metabolism *in vivo,* and hence most preferably exhibit a half-life *in vivo* of 60 to 240 minutes in humans. The imaging agent is preferably excreted *via* the kidney (i.e. exhibits urinary excretion). The imaging agent preferably exhibits a signal-to-background ratio at diseased foci of at least 1.5, most preferably at least 5, with at least 10 being especially preferred. Where the imaging agent comprises a radioisotope, clearance of one half of the peak level of imaging agent which is either non-specifically bound or free *in vivo,* preferably occurs over a time period less than or equal to the radioactive decay half-life of the radioisotope of the imaging moiety.

Furthermore, the molecular weight of the imaging agent is suitably up to 5000 Daltons. Preferably, the molecular weight is in the range 150 to 3000 Daltons, most preferably 200 to 1500 Daltons, with 300 to 800 Daltons being especially preferred.

An example of an imaging agent of the invention is illustrated below:

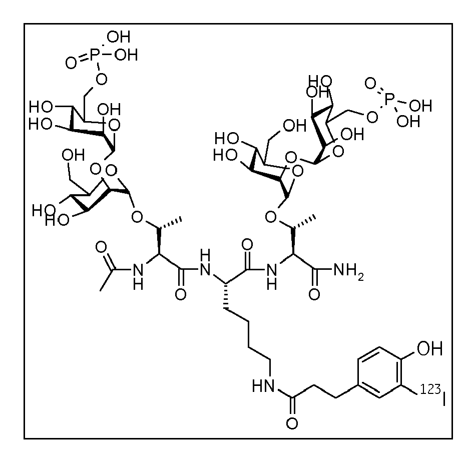

### Glycopeptide imaging agent 1

A synthetic route for obtaining the above compound is described in Example 1.

The imaging agents of this aspect of the invention are prepared from precursor compounds, which embody a further aspect of the invention and are described in more detail below.

In a further aspect, the present invention provides a method for the preparation of the imaging agent of the invention comprising reaction of a precursor with a suitable source of an imaging moiety wherein said precursor comprises:
(i) a vector with affinity for M6P receptor; and
(ii) a chemical group capable of reacting with a source of the imaging moiety so that the imaging moiety becomes attached to the compound to result in said imaging agent;
wherein said chemical group is either an integral part of said vector or is conjugated to said vector.

A "precursor" comprises a derivative of the vector, designed so that chemical reaction with a convenient chemical form of the imaging moiety occurs site-specifically; can be conducted in the minimum number of steps (ideally a single step); and without the need for significant purification (ideally no further purification), to give the desired imaging agent. Such precursors are synthetic and can conveniently be obtained in good chemical purity. The "precursor" may optionally comprise a protecting group for certain functional groups of the vector with affinity for M6P receptor.

By the term "protecting group" is meant a group which inhibits or suppresses undesirable chemical reactions, but which is designed to be sufficiently reactive that it may be cleaved from the functional group in question under mild enough conditions that do not modify the rest of the molecule. After deprotection the desired product is obtained. Protecting groups are well known to those skilled in the art and are suitably chosen from, for amine groups: Boc (where Boc is *tert*-butyloxycarbonyl), Fmoc (where Fmoc is fluorenylmethoxycarbonyll, trifluoroacetyl, allyloxycarbonyl, Dde [i.e. 1-(4,4-dimethyl-2,6-dioxocyclohexylidene)ethyll or Npys (i.e. 3-nitro-2-pyridine sulfenyl); and for carboxyl groups: methyl ester, *tert*-butyl ester or benzyl ester. For hydroxyl groups, suitable protecting groups are: methyl, ethyl or *tert-*butyl; alkoxymethyl or alkoxyethyl; benzyl; acetyl; benzoyl; trityl (Trt) or trialkylsilyl such as tetrabutyldimethylsilyl. For thiol groups, suitable protecting groups are: trityl and 4-methoxybenzyl. The use of further protecting groups are described in 'Protective Groups in Organic Synthesis', Theorodora W. Greene and Peter G. M. Wuts, (Third Edition, John Wiley & Sons, 1999).

Preferably, said chemical group capable of reacting with a source of an imaging moiety comprises:
(i) a chelator capable of complexing a metallic imaging moiety;
(ii) an organometallic derivative such as a trialkylstannane or a trialkylsilane;
(iii) a derivative containing an alkyl halide, alkyl tosylate or alkyl mesylate for nucleophilic substitution;
(iv) a derivative containing an aromatic ring activated towards nucleophilic or electrophilic substitution;
(v) a derivative containing a functional group which undergoes facile alkylation; or,
(vi) a derivative which alkylates thiol-containing compounds to give a thioether-containing product.

When the imaging moiety comprises a metal ion, the precursor comprises a chemical group capable of complexing the metal ion to form a metal complex. By the term "metal complex" is meant a coordination complex of the metal ion with one or more ligands. It is strongly preferred that the metal complex is "resistant to transchelation", i.e. does not readily undergo ligand exchange with other potentially competing ligands for the metal coordination sites. Potentially competing ligands include the vector for M6P receptor itself plus other excipients in the preparation *in vitro* (e.g. radioprotectants or antimicrobial preservatives used in the preparation), or endogenous compounds *in vivo* (e.g. glutathione, transferrin or plasma proteins).

Suitable ligands for use in the present invention which form metal complexes resistant to transchelation include: chelating agents, where 2-6, preferably 2-4, metal donor atoms are arranged such that 5- or 6-membered chelate rings result (by having a non-coordinating backbone of either carbon atoms or non-coordinating heteroatoms linking the metal donor atoms); or monodentate ligands which comprise donor atoms which bind strongly to the metal ion, such as isonitriles, phosphines or diazenides. Examples of donor atom types which bind well to metals as part of chelating agents are: amines, thiols, amides, oximes, and phosphines. Phosphines form such strong metal complexes that even monodentate or bidentate phosphines form suitable metal complexes. The linear geometry of isonitriles and diazenides is such that they do not lend themselves readily to incorporation into chelating agents, and are hence typically used as monodentate ligands. Examples of suitable isonitriles include simple alkyl isonitriles such as *tert*-butylisonitrile, and ether-substituted isonitriles such as mibi (i.e. 1-isocyano-2-methoxy-2-methylpropane). Examples of suitable phosphines include Tetrofosmin, and monodentate phosphines such as *tris*(3-methoxypropyl)phosphine. Examples of suitable diazenides include the HYNIC series of ligands i.e. hydrazine-substituted pyridines or nicotinamides.

Examples of suitable chelating agents for technetium which form metal complexes resistant to transchelation include, but are not limited to:
(i) diaminedioximes;
(ii) N₃S ligands having a thioltriamide donor set such as MAG₃ (mercaptoacetyltriglycine) and related ligands; or having a diamidepyridinethiol donor set such as Pica;
(iii) N₂S₂ ligands having a diaminedithiol donor set such as BAT or ECD (i.e. ethylcysteinate dimer), or an amideaminedithiol donor set such as MAMA;
(iv) N₄ ligands which are open chain or macrocyclic ligands having a tetramine, amidetriamine or diamidediamine donor set, such as cyclam, monoxocyclam or dioxocyclam; or,
(v) N₂O₂ ligands having a diaminediphenol donor set.

Preferred chelating agents of the invention for technetium are diaminedioximes and tetraamines, the preferred versions of which are now described in more detail.

Preferred diaminedioximes are of Formula (X): 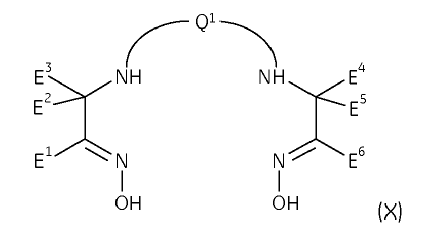 where E¹-E⁶ are each independently an R* group;
each R* is H or C₁₋₁₀ alkyl, C₃₋₁₀ alkylaryl, C₂₋₁₀ alkoxyalkyl, C₁₋₁₀ hydroxyalkyl, C₁₋₁₀ fluoroalkyl, C₂₋₁₀ carboxyalkyl or C₁₋₁₀ aminoalkyl, or two or more R* groups together with the atoms to which they are attached form a carbocyclic, heterocyclic, saturated or unsaturated ring, and wherein one or more of the R* groups is conjugated to the vector;
and Q¹ is a bridging group of formula -(J¹)_{f}- ;
where f is 3, 4 or 5 and each J¹ is independently -O-, -NR*- or -C(R*)₂- provided that -(J¹)_{f}-contains a maximum of one J¹ group which is -O- or -NR*-.

Preferred Q¹ groups are as follows:
Q¹ = -(CH₂)(CHR*)(CH₂)- i.e. propyleneamine oxime or PnAO derivatives;
Q¹ = -(CH₂)₂(CHR*)(CH₂)₂- i.e. pentyleneamine oxime or PentAO derivatives;
Q¹= -(CH₂)₂NR*(CH₂)₂-.
E¹ to E⁶ are preferably chosen from: C₁₋₃ alkyl, alkylaryl alkoxyalkyl, hydroxyalkyl, fluoroalkyl, carboxyalkyl or aminoalkyl. Most preferably, each E¹ to E⁶ group is CH₃.

The vector for M6P receptor is preferably conjugated at either the E¹ or E⁶ R* group, or an R* group of the Q¹ moiety. Most preferably, it is conjugated to an R* group of the Q¹ moiety. When it is conjugated to an R* group of the Q¹ moiety, the R* group is preferably at the bridgehead position. In that case, Q¹ is preferably -(CH₂)(CHR*)(CH₂)-, -(CH₂)₂(CHR*)(CH₂)₂- or -(CH₂)₂NR*(CH₂)₂-, most preferably -(CH₂)₂(CHR*)(CH₂)₂-. An especially preferred bifunctional diaminedioxime chelator has the Formula (Xa): 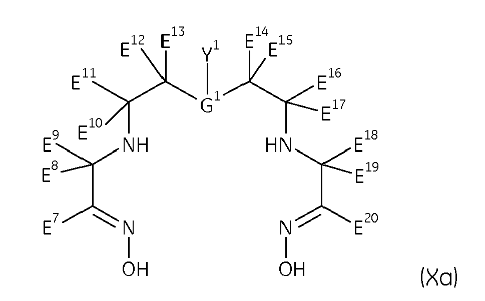 where:
E⁷-E²⁰ are each independently an R* group as defined above;
G¹ is N or CR*;
Y¹ is -(L⁶)ₛ-vector, wherein L⁶ and s are as defined for L¹ and n above, and 'vector' represents a vector with affinity for the M6P receptor as previously defined. Where there is a linker group -(L⁶)ₛ-, there is no other linker group joining the chelate and the vector.

A preferred chelator of Formula (Xa) is of Formula (Xb): 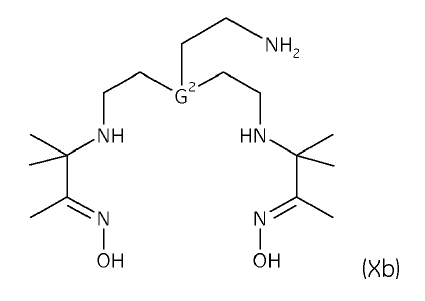 where G² is defined as for G¹ above, and is preferably CH (= "chelate X" for which the synthesis is described in Example 5);
such that the vector for M6P receptor is conjugated *via* the bridgehead -CH₂CH₂NH₂ group.

Preferred tetraamine chelators of the invention are of Formula Z: 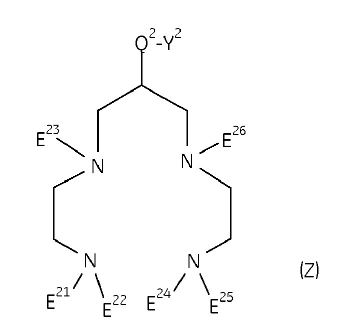 wherein:
Q² is a bridging group of formula -(J²)_{g}- ;
wherein g is 1-8 and each J² is independently -O-, -NR*- or -C(R*)₂-, preferably -C(R*)₂- and most preferably -CH₂-, wherein R* is as defined previously.

Y² is the group -(L⁷)ₜ-vector, wherein L⁷ and t are as previously defined for L¹ and n. Where there is a linker -(L⁷)ₜ-, no other linker joins the chelate to the vector. Preferably for these tetraamine chelates, the linker does not contain aryl rings. This helps to minimize the lipophilicity of the complex.

E²¹ to E²⁶ are an R* group as previously defined.

A most preferred tetraamine chelate of the present invention is of Formula Za: 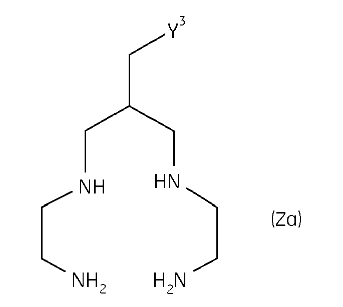 wherein Y³ is as defined above for Y².

An especially preferred tetraamine chelate of the present invention is of Formula Za wherein Y³ is -CO-vector ("chelate Z" synthesis of chelate without vector attached described in Example 2).

The above described ligands are particularly suitable for complexing technetium e.g. ^{94m}Tc or ^{99m}Tc, and are described more fully by Jurisson et al [Chem.Rev., 99, 2205-2218 (1999)]. The ligands are also useful for other metals, such as copper (⁶⁴Cu or ⁶⁷Cu), vanadium (e.g. ⁴⁸V), iron (eg. ⁵²Fe), or cobalt (e.g. ⁵⁵Co). Other suitable ligands are described in Sandoz WO 91/01144, which includes ligands which are particularly suitable for indium, yttrium and gadolinium, especially macrocyclic aminocarboxylate and aminophosphonic acid ligands. Examples of suitable chelating agents of this type include 1,4,7,10-tetraazacyclododecane-1,4,7,10-tetraacetic acid (DOTA) and diethylenetriaminepentaacetic acid (DTPA). Ligands which form non-ionic (i.e. neutral) metal complexes of gadolinium are known and are described in US 4885363. When the radiometal ion is technetium, the ligand is preferably a chelating agent which is tetradentate. Preferred chelating agents for technetium are the diaminedioximes, or those having an N₂S₂ or N₃S donor set as described above.

It is envisaged that the role of the linker group [defined above as either -(L⁶)ₛ- or -(L⁷)ₜ-] is to distance the relatively bulky metal complex, which results upon metal coordination, from the active site of the vector for M6P receptor so that e.g. receptor binding is not impaired. This can be achieved by a combination of flexibility (e.g. simple alkyl chains), so that the bulky group has the freedom to position itself away from the active site and/or rigidity such as a cycloalkyl or aryl spacer which orientates the metal complex away from the active site. The nature of the linker group can also be used to modify the biodistribution and excretion properties of the resulting metal complex of the conjugate. Thus, e.g. the introduction of ether groups in the linker will help to minimise plasma protein binding, or the use of polymeric linker groups such as polyalkyleneglycol, especially PEG (polyethyleneglycol) can help to prolong the lifetime of the agent in the blood *in vivo.*

Preferred linker groups -(L⁶)ₛ- or -(L⁷)ₜ- have a backbone chain which contains 2 to 10 atoms, most preferably 2 to 5 atoms, with 2 or 3 atoms being especially preferred. A minimum linker group backbone chain of 2 atoms confers the advantage that the chelator is well-separated from the biological targeting moiety so that any interaction is minimised. Furthermore, the vector is unlikely to compete effectively with the coordination of the chelator to the metal ion. In this way, both the biological targeting characteristics of the vector, and the metal complexing capability of the chelator is maintained. It is strongly preferred that the vector for M6P receptor is bound to the chelator in such a way that the linkage does not undergo facile metabolism in blood. That is because such metabolism would result in the imaging metal complex being cleaved off before the labelled vector for M6P receptor reaches the desired *in vivo* target site. The vector for M6P receptor is therefore preferably covalently bound to the metal complexes of the present invention *via* linker groups which are not readily metabolised. Suitable such linkages are carbon-carbon bonds, amide bonds, urea or thiourea linkages, or ether bonds.

Non-peptide linker groups such as alkylene groups or arylene groups have the advantage that there are no significant hydrogen bonding interactions with the conjugated vector for M6P receptor so that the linker does not wrap round onto the vector. Preferred alkylene spacer groups are -(CH₂)ᵤ- where u is an integer of value 2 to 5. Preferably q is 2 or 3. Preferred arylene spacers are of formula: 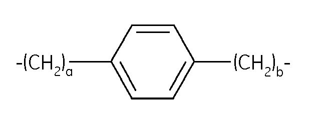 where: a and b are each independently 0, 1 or 2.

Preferred Y¹-Y³ groups are thus -CH₂CH₂-(L⁸)ᵥ-, - where v is an integer of value 0 to 3.

When the vector is a peptide, Y¹-Y³ is preferably -CH₂CH₂-(L⁹)_{w}- where L⁹ is -CO- or -NR"'-and w is 0 to 3, wherein R'" is as defined for R above. When either G¹ or G² is N and -(L⁹)_{w}- is -NH-, this grouping has the additional advantage that it stems from the symmetrical intermediate N(CH₂CH₂NH₂)₃, which is commercially available.

When the imaging metal is technetium, the usual technetium starting material is pertechnetate, i.e. TcO₄⁻ which is technetium in the Tc(VII) oxidation state. Pertechnetate itself does not readily form metal complexes, hence the preparation of technetium complexes usually requires the addition of a suitable reducing agent such as stannous ion to facilitate complexation by reducing the oxidation state of the technetium to the lower oxidation states, usually Tc(I) to Tc(V). The solvent may be organic or aqueous, or mixtures thereof. When the solvent comprises an organic solvent, the organic solvent is preferably a biocompatible solvent, such as ethanol or DMSO. Preferably the solvent is aqueous, and is most preferably isotonic saline.

Where the imaging moiety is radioiodine, preferred precursors are those which comprise a derivative which either undergoes electrophilic or nucleophilic iodination or undergoes condensation with a labelled aldehyde or ketone. Examples of the first category are:
(a) organometallic derivatives such as a trialkylstannane (eg. trimethylstannyl or tributylstannyl), or a trialkylsilane (eg. trimethylsilyl) or an organoboron compound (eg. boronate esters or organotrifluoroborates);
(b) a non-radioactive alkyl bromide for halogen exchange or alkyl tosylate, mesylate or triflate for nucleophilic iodination;
(c) aromatic rings activated towards electrophilic iodination (eg. phenols) and aromatic rings activated towards nucleophilic iodination (eg. aryl iodonium salt aryl diazonium, aryl trialkylammonium salts or nitroaryl derivatives).

The precursor preferably comprises: a non-radioactive halogen atom such as an aryl iodide or bromide (to permit radioiodine exchange); an activated precursor aryl ring (e.g. a phenol group); an organometallic precursor compound (e.g. trialkyltin, trialkylsilyl or organoboron compound); or an organic precursor such as triazenes or a good leaving group for nucleophilic substitution such as an iodonium salt. Preferably for radioiodination, the precursor comprises an organometallic precursor compound, most preferably trialkyltin.

Precursors and methods of introducing radioiodine into organic molecules are described by Bolton [J.Lab.Comp.Radiopharm., 45, 485-528 (2002)]. Precursors and methods of introducing radioiodine into proteins are described by Wilbur [Bioconj.Chem., 3(6), 433-470 (1992)]. Suitable boronate ester organoboron compounds and their preparation are described by Kabalaka et al [Nucl.Med.Biol., 29, 841-843 (2002) and 30, 369-373(2003)]. Suitable organotrifluoroborates and their preparation are described by Kabalaka et al [Nucl.Med.Biol., 31, 935-938 (2004)].

Examples of aryl groups to which radioactive iodine can be attached are given below:

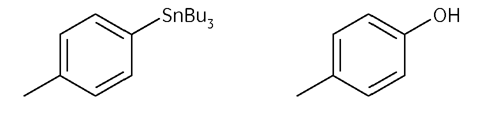

Both contain substituents which permit facile radioiodine substitution onto the aromatic ring. Alternative substituents containing radioactive iodine can be synthesised by direct iodination *via* radiohalogen exchange, e.g.

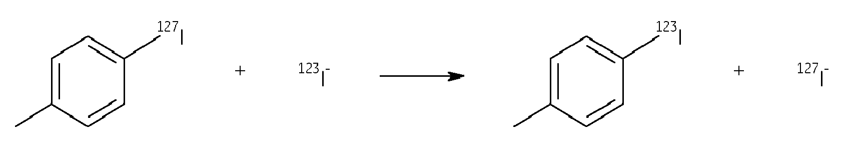

The radioiodine atom is preferably attached *via* a direct covalent bond to an aromatic ring such as a benzene ring, or a vinyl group since it is known that iodine atoms bound to saturated aliphatic systems are prone to *in vivo* metabolism and hence loss of the radioiodine.

When the imaging moiety is a radioactive isotope of fluorine the radiofluorine atom may form part of a fluoroalkyl or fluoroalkoxy group, since alkyl fluorides are resistant to *in vivo* metabolism. Alternatively, the radiofluorine atom may be attached *via* a direct covalent bond to an aromatic ring such as a benzene ring. Radiohalogenation may be carried out *via* direct labelling using the reaction of ¹⁸F-fluoride with a suitable chemical group in the precursor having a good leaving group, such as an alkyl bromide, alkyl mesylate or alkyl tosylate. ¹⁸F can also be introduced by alkylation of N-haloacetyl groups with a ¹⁸F(CH₂)₃OH reactant, to give -NH(CO)CH₂O(CH₂)₃¹⁸F derivatives. For aryl systems, ¹⁸F-fluoride nucleophilic displacement from an aryl diazonium salt, aryl nitro compound or an aryl quaternary ammonium salt are suitable routes to aryl-¹⁸F derivatives.

A further approach for radiofluorination as described in WO 03/080544, is to react a precursor compound comprising one of the following substituents: 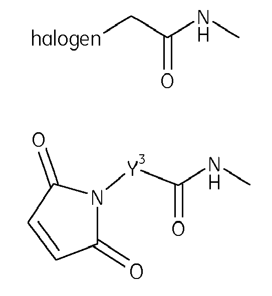 with a compound of Formula V:

¹⁸F-Y⁴-SH (V)

wherein Y³ is a linker of formula -(L¹⁰)ₓ- wherein L¹⁰ is as previously defined for L¹, x is 1-10 and optionally includes 1-6 heteroatoms; and,
Y⁴ is a linker of formula -(L¹¹)_{y}- wherein L¹¹ is as previously defined for L¹, y is 1-30 and
optionally includes 1 to 10 heteroatoms;
to give radiofluorinated imaging agents of formula (Va) or (Vb) respectively: 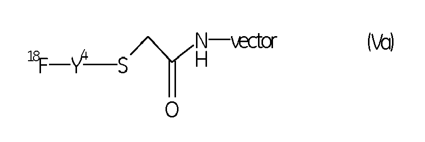 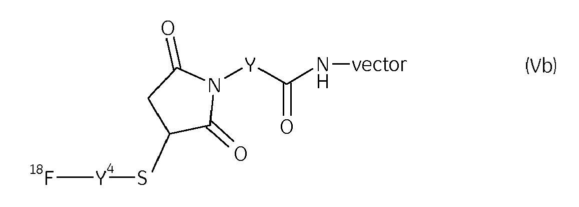
wherein Y⁴ is as defined above, and 'vector' is a vector with affinity for the M6P receptor, as defined above in relation to the imaging agent of the invention.

Further details of synthetic routes to ¹⁸F-labelled derivatives are described by Bolton, J.Lab.Comp.Radiopharm., 45, 485-528 (2002).

A ¹⁸F-labelled compound of the invention may be obtained by formation of ¹⁸F fluorodialkylamines and subsequent amide formation when the ¹⁸F fluorodialkylamine is reacted with a precursor containing, e.g. chlorine, P(O)Ph₃ or an activated ester.

The imaging moiety may for example be incorporated into the diphosphorylated glycopeptide *via* one of the amino acid residues present in the peptide linking the M6P units to form precursor compounds such as:
(iii) Ac-Thr[α-D-M6P-(1,2)-α-D-mannose]-Lys(chelate)-Thr[α-D-M6P-(1,2)-α-D-mannose]-NH₂ ("glycopeptide precursor 1")
(iv) Ac-Thr[α-D-M6P]-Gly-Lys-(chelate)-Gly-Thr[α-D-M6P]-NH₂ ("glycopeptide precursor 2")

In the case of combination vectors, any of the elements may be derivatised *via* suitable groups, as discussed above, that are available for derivatisation in order to form precursors.

In a preferred embodiment, the precursor of the invention is in sterile, apyrogenic form. The precursor can accordingly be used for the preparation of a pharmaceutical composition and is also suitable for inclusion as a component in a kit for the preparation of a pharmaceutical composition. These aspects are discussed in more detail below in relation to additional aspects of the invention.

In a further preferred embodiment the precursor of the invention is bound to a solid phase. The precursor is preferably supplied covalently attached to a solid support matrix in such a way that the labeling reaction results in simultaneous labeling and cleavage from the solid phase. The desired imaging agent product therefore forms in solution and any starting materials and impurities remain bound to the solid phase. As an example of such a system, precursors for solid phase electrophilic fluorination with ¹⁸F-fluoride are described in WO 03/002489, and precursors for solid phase nucleophilic fluorination with ¹⁸F-fluoride are described in WO 03/002157. A cartridge may be provided, preferably as part of a kit, which can be plugged into a suitably adapted automated synthesizer. The cartridge may contain, apart from the solid support- bound precursor, a column to remove unwanted fluoride ion, and an appropriate vessel connected so as to allow the reaction mixture to be evaporated and allow the product to be formulated as required.

A further aspect of the present invention is a precursor as defined in relation to the method of preparation of the imaging agent, wherein said chemical group preferably:
(i) comprises a chelator capable of complexing a metallic imaging moiety;
(ii) comprises an organometallic derivative such as a trialkylstannane or a trialkylsilane;
(iii) comprises a derivative containing an alkyl halide, alkyl tosylate or alkyl mesylate for nucleophilic substitution; or,
(iv) comprises a derivative which alkylates thiol-containing compounds to give a thioether-containing product.

In a further aspect, the present invention provides a pharmaceutical composition which comprises the imaging agent as described above, together with a biocompatible carrier, in a form suitable for mammalian administration. Preferably, the pharmaceutical composition is a radiopharmaceutical composition.

The "biocompatible carrier" is a fluid, especially a liquid, in which the imaging agent is suspended or dissolved, such that the composition is physiologically tolerable, i.e. can be administered to the mammalian body without toxicity or undue discomfort. The biocompatible carrier medium is suitably an injectable carrier liquid such as sterile, pyrogen-free water for injection; an aqueous solution such as saline (which may advantageously be balanced so that the final product for injection is either isotonic or not hypotonic); an aqueous solution of one or more tonicity-adjusting substances (e.g. salts of plasma cations with biocompatible counterions), sugars (e.g. glucose or sucrose), sugar alcohols (e.g. sorbitol or mannitol), glycols (e.g. glycerol), or other non-ionic polyol materials (e.g. polyethyleneglycols, propylene glycols and the like). The biocompatible carrier medium may also comprise biocompatible organic solvents such as ethanol. Such organic solvents are useful to solubilise more lipophilic compounds or formulations. Preferably the biocompatible carrier medium is pyrogen-free water for injection, isotonic saline or an aqueous ethanol solution. The pH of the biocompatible carrier medium for intravenous injection is suitably in the range 4.0 to 10.5.

Such pharmaceutical compositions are suitably supplied in either a container which is provided with a seal which is suitable for single or multiple puncturing with a hypodermic needle (e.g. a crimped-on septum seal closure) whilst maintaining sterile integrity. Such containers may contain single or multiple patient doses. Preferred multiple dose containers comprise a single bulk vial (e.g. of 10 to 30 cm³ volume) which contains multiple patient doses, whereby single patient doses can thus be withdrawn into clinical grade syringes at various time intervals during the viable lifetime of the preparation to suit the clinical situation. Pre-filled syringes are designed to contain a single human dose, or "unit dose" and are therefore preferably a disposable or other syringe suitable for clinical use. Where the pharmaceutical composition is a radiopharmaceutical composition, the pre-filled syringe may optionally be provided with a syringe shield to protect the operator from radioactive dose. Suitable such radiopharmaceutical syringe shields are known in the art and preferably comprise either lead or tungsten.

The pharmaceutical composition of the present invention may be prepared from a kits, as is described in a further aspect of the invention, below. Alternatively, the pharmaceutical composition may be prepared under aseptic manufacture conditions to give the desired sterile product. The pharmaceutical composition may also be prepared under non-sterile conditions, followed by terminal sterilisation using e.g. gamma-irradiation, autoclaving, dry heat or chemical treatment (e.g. with ethylene oxide). Preferably, the pharmaceutical composition of the present invention is prepared from a kit.

As described above in relation to the imaging agent of the invention, for radiopharmaceutical compositions the most preferred radioactive imaging moieties of the invention are ^{99m}Tc, ¹²³I and ¹⁸F_{.}

In a yet further aspect, the present invention provides kits for the preparation of the pharmaceutical compositions of the invention. Such kits comprise a suitable precursor of the invention, preferably in sterile non-pyrogenic form, so that reaction with a sterile source of an imaging moiety gives the desired pharmaceutical composition with the minimum number of manipulations. Such considerations are particularly important for radiopharmaceuticals, especially where the radioisotope has a relatively short half-life, and for ease of handling and hence reduced radiation dose for the radiopharmacist. Hence, the reaction medium for reconstitution of such kits is preferably a "biocompatible carrier" as defined above, and is most preferably aqueous.

Suitable kit containers comprise a sealed container which permits maintenance of sterile integrity and/or radioactive safety, plus optionally an inert headspace gas (e.g. nitrogen or argon), whilst permitting addition and withdrawal of solutions by syringe. A preferred such container is a septum-sealed vial, wherein the gas-tight closure is crimped on with an overseal (typically of aluminium). Such containers have the additional advantage that the closure can withstand vacuum if desired e.g. to change the headspace gas or degas solutions.

Preferred aspects of the precursor when employed in the kit are as described above in relation to the method of synthesis of the imaging agents of the invention. The precursors for use in the kit may be employed under aseptic manufacture conditions to give the desired sterile, non-pyrogenic material. The precursors may also be employed under non-sterile conditions, followed by terminal sterilisation using e.g. gamma-irradiation, autoclaving, dry heat or chemical treatment (e.g. with ethylene oxide). Preferably, the precursors are employed in sterile, non-pyrogenic form. Most preferably the sterile, non-pyrogenic precursors are employed in the sealed container as described above.

The precursor of the kit is preferably supplied covalently attached to a solid support matrix as described above in relation to the method of synthesis of the imaging agents of the invention.

For ^{99m}Tc, the kit is preferably lyophilised and is designed to be reconstituted with sterile ^{99m}Tc-pertechnetate (TcO₄⁻) from a ^{99m}Tc radioisotope generator to give a solution suitable for human administration without further manipulation. Suitable kits comprise a container (e.g. a septum-sealed vial) containing the uncomplexed chelating agent, together with a pharmaceutically acceptable reducing agent such as sodium dithionite, sodium bisulphite, ascorbic acid, formamidine sulphinic acid, stannous ion, Fe(II) or Cu(I); together with at least one salt of a weak organic acid with a biocompatible cation. By the term "biocompatible cation" is meant a positively charged counterion which forms a salt with an ionised, negatively charged group, where said positively charged counterion is also non-toxic and hence suitable for administration to the mammalian body, especially the human body. Examples of suitable biocompatible cations include: the alkali metals sodium or potassium; the alkaline earth metals calcium and magnesium; and the ammonium ion. Preferred biocompatible cations are sodium and potassium, most preferably sodium.

The kits for preparation of ^{99m}Tc imaging agents may optionally further comprise a second, weak organic acid or salt thereof with a biocompatible cation, which functions as a transchelator. The transchelator is a compound which reacts rapidly to form a weak complex with technetium, then is displaced by the chelator of the kit. This minimises the risk of formation of reduced hydrolysed technetium (RHT) due to rapid reduction of pertechnetate competing with technetium complexation. Suitable such transchelators are the weak organic acids and salts thereof described above, preferably tartrates, gluconates, glucoheptonates, benzoates, or phosphonates, preferably phosphonates, most especially diphosphonates. A preferred such transchelator is MDP, ie. methylenediphosphonic acid, or a salt thereof with a biocompatible cation.

As an alternative to use of a chelator in free form, the kit for preparation of ^{99m}Tc imaging agents may optionally contain a non-radioactive metal complex of the chelator which, upon addition of the technetium, undergoes transmetallation (i.e. ligand exchange) giving the desired product. Suitable such complexes for transmetallation are copper or zinc complexes.

The pharmaceutically acceptable reducing agent used in the kit is preferably a stannous salt such as stannous chloride, stannous fluoride or stannous tartrate, and may be in either anhydrous or hydrated form. The stannous salt is preferably stannous chloride or stannous fluoride.

The kits may optionally further comprise additional components such as a radioprotectant, antimicrobial preservative, pH-adjusting agent or filler.

By the term "radioprotectant" is meant a compound which inhibits degradation reactions, such as redox processes, by trapping highly-reactive free radicals, such as oxygen-containing free radicals arising from the radiolysis of water. The radioprotectants of the present invention are suitably chosen from: ascorbic acid, para-aminobenzoic acid (i.e. 4-aminobenzoic acid), gentisic acid (i.e. 2,5-dihydroxybenzoic acid) and salts thereof with a biocompatible cation. The "biocompatible cation" and preferred embodiments thereof are as described above.

By the term "antimicrobial preservative" is meant an agent which inhibits the growth of potentially harmful micro-organisms such as bacteria, yeasts or moulds. The antimicrobial preservative may also exhibit some bactericidal properties, depending on the dose. The main role of the antimicrobial preservative(s) of the present invention is to inhibit the growth of any such micro-organism in the pharmaceutical composition post-reconstitution, i.e. in the imaging agent product itself. The antimicrobial preservative may, however, also optionally be used to inhibit the growth of potentially harmful micro-organisms in one or more components of the kit prior to reconstitution. Suitable antimicrobial preservative(s) include: the parabens, i.e. methyl, ethyl, propyl or butyl paraben or mixtures thereof; benzyl alcohol; phenol; cresol; cetrimide and thiomersal. Preferred antimicrobial preservative(s) are the parabens.

The term "pH-adjusting agent" means a compound or mixture of compounds useful to ensure that the pH of the reconstituted kit is within acceptable limits (approximately pH 4.0 to 10.5) for human or mammalian administration. Suitable such pH-adjusting agents include pharmaceutically acceptable buffers, such as tricine, phosphate or TRIS [ie. *tris*(hydroxymethyl)aminomethane], and pharmaceutically acceptable bases such as sodium carbonate, sodium bicarbonate or mixtures thereof. When the conjugate is employed in acid salt form, the pH adjusting agent may optionally be provided in a separate vial or container, so that the user of the kit can adjust the pH as part of a multi-step procedure.

By the term "filler" is meant a pharmaceutically acceptable bulking agent which may facilitate material handling during production and lyophilisation. Suitable fillers include inorganic salts such as sodium chloride, and water soluble sugars or sugar alcohols such as sucrose, maltose, mannitol or trehalose.

The imaging agents of the invention are useful for *in vivo* imaging. Accordingly, in an additional aspect, the present invention provides an imaging agent of the invention for use in an *in vivo* diagnostic or imaging method, e.g. SPECT or PET. Preferably said method relates to the *in vivo* imaging of a condition in which the M6P receptor is upregulated and therefore has utility in the diagnosis of conditions associated with fibrosis such as liver fibrosis, congestive heart failure, glomerulosclerosis and respiratory failure. In a most preferred embodiment, said condition is liver fibrosis, where the M6P receptor is known to be upregulated on HSC and on liver parenchymal cells.

This aspect of the invention also provides a method for the *in vivo* diagnosis or imaging in a subject of a condition in which the M6P receptor is upregulated, comprising administration of the pharmaceutical composition of the invention, described above. Said subject is preferably a mammal and most preferably a human. In an alternative embodiment, this aspect of the invention furthermore provides for the use of the imaging agent of the invention for imaging *in vivo* in a subject of a condition in which the M6P receptor is upregulated wherein said subject is previously administered with the pharmaceutical composition of the invention. By "previously administered" is meant that the step involving the clinician, wherein the pharmaceutical is given to the patient e.g., intravenous injection, has already been carried out.

In another further aspect the invention provides a method of monitoring the effect of treatment of a human or animal body with a drug to combat a condition in which the M6P receptor is upregulated, said method comprising administering to said body an imaging agent of the invention and detecting the uptake of said imaging agent, said administration and detection optionally but preferably being effected repeatedly, e.g. before, during and after treatment with said drug.

### Brief Description of the Examples

Example 1 provides the synthesis of ¹²³I-labelled glycopeptide imaging agent 1.

### Examples

### List of abbreviations used in Example

- HPLC: high-performance liquid chromatography
- TFA: trifluoroacetic acid

### Example 1: Synthesis of ¹²³I-labelled Glycopeptide Imaging Agent 1.

The precursor of Glycopeptide Imaging Agent 1 is shown below:

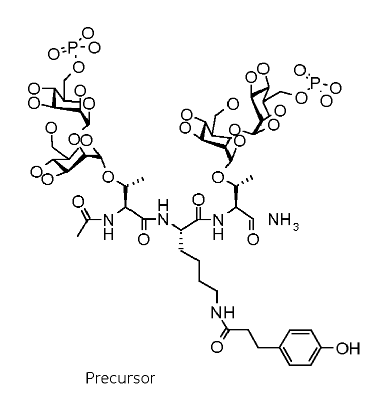

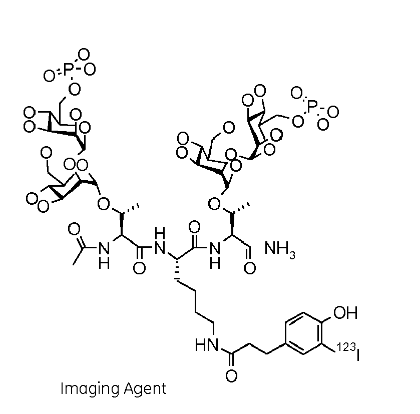

The precursor was radioiodinated as follows: 10 µl of ¹²⁷NaI (15 mg/100 ml in 0.01M NaOH) solution (containing 1 x 10⁻⁸ moles ¹²⁷NaI) was initially added to 200µl ammonium acetate buffer (0.2 M, pH 4) in a microcentrifuge tube. The ¹²⁷NaI /NH₄OAc solution was then added to ¹²³I-NaI in 0.05 M NaOH (from GE Healthcare) containing 10mCi (364 mCi/ml) of activity and the solution was mixed well. 5 µl of peracetic acid (36-40 wt % solution in acetic acid) was added to 5 ml H₂O, mixed well and 10 µl of the diluted solution was added to the ¹²³/¹²⁷I-NaI reaction mixture.

100 µl of precursor (0.74mM solution in H₂O) was added to the reaction mixture, mixed with a pipette and the reaction was analysed after 5 minutes. The product was analysed or purified by HPLC using a Phenomenex Luna column 5u, C₁₈(2) 100A, 150 x 4.6 mm.
Solvent A-0.1% TFA in H₂O
Solvent B - 0.1 % TFA in acetonitrile
0 min - 100% solvent A, 0% solvent B
20 min - 50% solvent A, 50% solvent B
25 min - 10% solvent A, 90% solvent B
35 min - 10% solvent A, 90% solvent B
36 min - 100% solvent A, 0% solvent B
43 min - 100% solvent A, 0% solvent B

The retention time of M6P Imaging Agent 1 under these conditions is 9.9 min.

## Claims

1. An imaging agent comprising:
(i) a vector with affinity for the mannose-6-phosphate (M6P) receptor; and,
(ii) an imaging moiety
wherein the imaging moiety is present either as an integral part of the vector or the imaging moiety is conjugated to the vector *via* a suitable chemical group;
and wherein said vector comprises a diphosphorylated glycopeptide.

2. The imaging agent of claim 1 wherein said vector is a diphosphorylated glycopeptide and is of Formula 1: 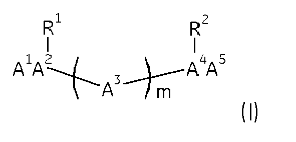 wherein
R¹ and R² are independently selected from:
(i) a natural L- or D-monosaccharide chosen from: glucose, mannose, galactose, fucose, rhammanose, N-acetylglucosamine, N-acetylgalactosaminyl, fructose and N-acetylneuraminic acid, or phosphorylated or sulphated versions thereof; or,
(ii) an oligosaccharide composed of monosaccharides selected from (i);
A¹ and A⁵ are independently selected from the group consisting of -H, -OH, -NH₂, - acetyl, D- or L-amino acids, peptides, glycopeptides, peptidomimetics and oligonucleotides,
A² and A⁴ are independently selected from the group of D- or L-hydroxy amino acids, e.g. Ser, Thr, Hyl, Hyp, Tyr or D- or L-carboxamido amino acids, e.g. Asn and Gln, and A³ is selected from the group of genetically encoded or non-encoded amino acids in their D- or L-form or peptidomimetics or nucleotides,
and wherein m is an integer between 1 and 30;
and wherein any residue in the linear sequence A¹ - A⁵ may be covalently linked to form a cyclic compound.

3. The imaging agent of claim 2 wherein the diphosphorylated glycopeptide is
(i) Ac-Thr[α-D-M6P-(1,2)-α-D-mannose]-Lys(aminobenzamide)-Thr[α-D-M6P-(1,2)-α-D-mannose]-NH₂
(ii) Ac-Thr[α-D-M6P]-Gly-Lys-Gly-Thr[α-D-M6P]-NH₂

4. The imaging agent of claim 2 wherein said vector is a multivalent targeting vector combining two or more of the vectors of claims 1-3.

5. The imaging agent of any one of claims 1-4 wherein said imaging moiety is selected from:
(i) a radioactive metal ion;
(ii) a paramagnetic metal ion;
(iii) a gamma-emitting radioactive halogen;
(iv) a positron-emitting radioactive non-metal;
(v) a hyperpolarised NMR-active nucleus;
(vi) a reporter suitable for *in vivo* optical imaging; and
(vii) a β-emitter suitable for intravascular detection.

6. A method for the preparation of the imaging agent of any of claims 1-5 comprising reaction of a precursor with a suitable source of an imaging moiety wherein said precursor comprises:
(i) a vector with affinity for M6P receptor as defined in claims 1 to 3; and
(ii) a chemical group capable of reacting with a source of the imaging moiety so that the imaging moiety becomes attached to the compound to result in said imaging agent;
wherein said chemical group is either an integral part of said vector or is conjugated to said vector.

7. The method according to claim 6 wherein said chemical group:
(i) comprises a chelator capable of complexing a metallic imaging moiety;
(ii) comprises an organometallic derivative such as a trialkylstannane or a trialkylsilane;
(iii) comprises a derivative containing an alkyl halide, alkyl tosylate or alkyl mesylate for nucleophilic substitution;
(iv) comprises a derivative containing an aromatic ring activated towards nucleophilic or electrophilic substitution;
(v) comprises a derivative containing a functional group which undergoes facile alkylation; or,
(vi) comprises a derivative which alkylates thiol-containing compounds to give a thioether-containing product

8. A precursor as defined in the method of any of claims 6-7 wherein said chemical group:
(i) comprises a chelator capable of complexing a metallic imaging moiety;
(ii) comprises an organometallic derivative such as a trialkylstannane or a trialkylsilane;
(iii) comprises a derivative containing an alkyl halide, alkyl tosylate or alkyl mesylate for nucleophilic substitution;
(iv) comprises a derivative which alkylates thiol-containing compounds to give a thioether-containing product

9. A pharmaceutical composition comprising the imaging agent of any one of claims 1-5, together with a biocompatible carrier in a form suitable for mammalian administration.

10. The pharmaceutical composition of claim 9 wherein said imaging agent comprises a radioactive imaging moiety.

11. A kit for the preparation of the pharmaceutical composition of any of claims 9-10.

12. An imaging agent of any of claims 1-5 for use in an *in vivo* diagnostic or imaging method.

13. An imaging agent of any of claims 1-5 for use in a method of monitoring the effect of treatment of a human or animal body with a drug to combat a condition in which the M6P receptor is upregulated, said method comprising administering to said body the imaging agent of any of claims 1-5 and detecting the uptake of said imaging agent.
